# EUROPEAN PATENT SPECIFICATION

(11) **EP 1 782 850 B1**
(45) Date of publication and mention of the grant of the patent: **11.07.2012**
(21) Application number: 06255580.0
(22) Date of filing: 30.10.2006
(51) Int. Cl.: A61L 29/08, A61M 25/00

(54) **Method of removing a stylette from a catheter**
Verfahren zur Entfernung eines Stylets aus einem Katheter
Méthode pour retirer un stylet d'un cathéter

(30) Priority: 31.10.2005 US 262697
(43) Date of publication of application: 09.05.2007
(73) Proprietor: CODMAN & SHURTLEFF INC., Rynham, MA 02767 (US)
(72) Inventor: Venugopalan, Ramakrishna, Holliston, MA 01746 (US); Schorn, Greg, Milford, MA 01757 (US)
(74) Representative: Tunstall, Christopher Stephen

(56) References cited:
- WO-A-2004/093968
- US-A- 4 938 746
- US-A- 5 357 961
- US-A- 5 823 961
- US-A1- 2004 002 693

## Description

### BACKGROUND OF THE INVENTION

### 1. Field of the Invention

The present invention relates to methods of removing a stylette from a catheter. More particularly, the present invention relates to methods of removing a stylette from a catheter with the use of a hydrophilic coated stylette. The invention further relates to combinations of a catheter, a hydrophilic coated stylette and a syringe.

### 2. Discussion of Related Art

It is well known in the art to place a catheter at a desired target site within the body. To aid in inserting the catheter, which often has to tunnel through muscle and tissue, a stylette or guide wire (hereinafter referred to as a stylette) may be used to help provide rigidity during the insertion process. Once the catheter is placed at a desired site, the stylette is removed. But in this process, it is sometimes difficult to remove the stylette. The stylette sticks to the catheter and can cause, among other things, displacement of the distal end of the catheter from the target site, and/or cutting of the catheter.

Some have attempted to solve this problem by coating the stylette with polytetrafluoroethylene ("PTFE") to ease its removal. However, users continue to be dissatisfied with this attempted solution because the stylette still sometimes sticks to the catheter.

Published U.S. Patent Application No. 2005/0100580, entitled Hydrophilic Coated Medical Device teaches that a hydrophilic coating allows for easy insertion of wire guides during cardiac catherization. However, there is no teaching or suggestion of coating a guide wire with a hydrophilic material to aid in the removal of a stylette after a catheter has been tunneled into place at a desired site within a body.

Thus, there is a need in the art for a method of more effectively removing a stylette from a catheter, especially after the catheter has been placed at a desired site within a body.

US 5,823,961 and US 5,357,961, each disclose a combination of a catheter having proximal and distal ends and an inner surface, a hydrophilic coated stylette within the catheter, and a syringe connected to the proximal end of the catheter for wetting the hydrophilic coated stylette.

The catheter of US 5,823,961 may be coated with chemical substrates to reduce surface friction between the catheter and the stylette.

US 4,938,746 and US 2004/0002693 each disclose a combination of a catheter having proximal and distal ends and an inner surface, a hydrophilic coated stylette within the catheter, and means for wetting the hydrophilic coated stylette.

### SUMMARY OF THE INVENTION

According to a first aspect of the invention, there is provided a method as set forth in the accompanying claim 1.

According to a second aspect of the invention, there is provided a method as set forth in the acccompanying claim 2.

According to a third aspect of the invention, there is provided a combination as set forth in the accompanying claim 10.

According to a fourth aspect of the invention, there is provided a combination as set forth in the accompanying claim 11.

In accordance with a currently preferred exemplary embodiment, the present invention involves a hydrophilic coated stylette that is pre-loaded in an implantable catheter. The hydrophilic coated stylette is wetted with saline to activate the hydrophilic coating. The stylette loaded catheter is tunneled, typically through muscle and tissue, to a desired target site within the body. The hydrophilic coated stylette is then removed from the catheter, while maintaining the catheter intact. The proximal end of the catheter can be connected to an outlet of an implantable infusion pump so that medicament can be delivered directly to the target site.

### BRIEF DESCRIPTION OF THE DRAWINGS

The above and still further objects, features and advantages of the present invention will become apparent upon consideration of the following detailed description of a specific embodiment thereof, especially when taken in conjunction with the accompanying drawings wherein like reference numerals in the various figures are utilized to designate like components, and wherein:
Figure 1 is a perspective view of a hydrophilic coated stylette loaded in a catheter with a syringe connected to a proximal end of the catheter;
Figure 2 is a cross-sectional view taken along line 2-2 of Fig. 1 and looking in the direction of the arrows; and
Figure 3 is a cross-sectional view taken along line 3-3 of Fig. 2 and looking in the direction of the arrows.

### DETAILED DESCRIPTION OF THE CURRENTLY PREFERRED EXEMPLARY EMBODIMENT

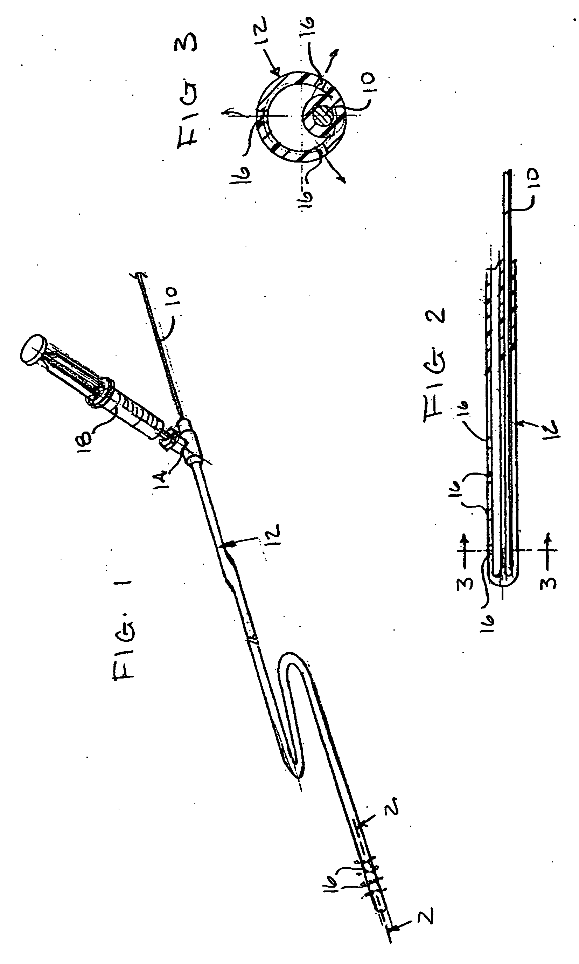

Referring now to Figs. 1-3, a device and method for removing a hydrophilic coated stylette 10 from an implantable catheter 12 is illustrated. Catheter 12 is, in a currently preferred embodiment, an intraspinal catheter. As shown in Figs. 1-3, catheter 12 is preloaded with a hydrophilic coated stylette 10. Thus, the hydrophilic coated stylette 10 is placed within catheter 12. A syringe connecter 14, preferably in the form of a luer lock, is connected to the proximal end of catheter 12. A distal end of catheter 12 has at least one opening 16 so that medicament can be delivered to the spine via the catheter. In a currently preferred embodiment, the catheter is pre-loaded with stylette 10, and delivered in this state to the end user.

In a currently preferred embodiment, the end user will connect a syringe 18 to the proximal end of catheter 12 via the luer lock as shown in Fig. 1. Syringe 18 preferably contains pure saline. The saline is injected through the catheter 12 to hydrate the hydrophilic stylette 10. The user can confirm that the stylette is fully wetted by witnessing droplets of saline exiting from distal openings 16, as illustrated in Fig. 1.

The stylette loaded and wetted catheter is then placed at a desired target site within the body. The stylette provides rigidity to the catheter to aid in inserting the catheter, which often has to tunnel through muscle and tissue, at the desired target site. In a currently preferred embodiment, the target site is intraspinal. Once the distal end of catheter 12 is placed at the target site, the hydrophilic coated stylette 10 can be removed from catheter 12. Because the stylette has recently been hydrated, stylette 10 can be removed while maintaining the catheter intact. Thus, the present invention reduces the risk that the location of the distal end of the catheter will be inadvertently displaced, or that the catheter will be accidentally cut during the stylette removal process. Catheter 12 can now be fluidly connected to a medicinal product to deliver the medicinal product to the target site. For example, the proximal end of catheter 12 can be connected to an outlet of an implantable infusion pump so that medicament can be delivered directly to the target site. Of course, one skilled in the art will readily recognize that other devices may be used to deliver medicament to the catheter.

In a currently preferred embodiment, catheter 12 is made of a polymeric material. More preferably, catheter 12 is made of silicone. However, catheter 12 could also be made of polyurethane or other similar biocompatible material as those skilled in the art will readily recognize. Catheter 12 may be reinforced with a structural member that is stiffer than the polymeric material, whether it be silicone, polyurethane or other similar biocompatible material. The reinforce catheter could be made individually or by using a combination of a polymer or metal/alloy. Catheter 12 has an inner surface that is coated with polytetrafluoroethylene or a hydrophilic material. If the catheter inner surface is coated with a hydrophilic material that hydrophilic material may be the same as the hydrophilic material coated on the stylette. Alternatively, if the catheter inner surface is coated with a hydrophilic material that hydrophilic material may be different from the hydrophilic material coated on the stylette, which when wetted in combination provides added lubricity.

Having described the presently preferred exemplary embodiment of a method of removing a stylette from a catheter in accordance with the present invention, it is believed that other modifications, variations and changes will be suggested to those skilled in the art in view of the teachings set forth herein. Substitutions of elements from one described embodiment to another are also fully intended and contemplated. It is also to be understood that the drawings are not necessarily drawn to scale, but that they are merely conceptual in nature. It is, therefore, to be understood that all such modifications, variations, and changes are believed to fall within the scope of the present invention as defined by the appended claims.

## Claims

1. A method of removing A hydrophilic coated stylette (10) from an implantable catheter (12), said method comprising the steps of:
providing an implantable catheter (12) having an inner surface that is coated with polytetrafluoroethylene;
placing the hydrophilic coated stylette (10) within the catheter (12);
wetting the hydrophilic coated stylette (10); and
removing the hydrophilic coated stylette (10) from the catheter (12).

2. A method of removing a hydrophilic coated stylette (10) from an implantable catheter (12), said method comprising the steps of:
providing an implantable catheter (12) having an inner surface that is coated with a hydrophilic material;
placing the hydrophilic coated stylette (10) within the catheter (12);
wetting the hydrophilic coated stylette (10); and
removing the hydrophilic coated stylette (10) from the catheter (12).

3. The method according to claim 1 or claim 2, wherein the wetting step occurs after the placing the hydrophilic coated stylette (10) within the catheter (12) step.

4. The method according to claim 1 or claim 2, wherein the removing step occurs after the wetting step.

5. The method according to claim 1 or claim 2, wherein the catheter (12) is made of a polymeric material, such as silicone or polyurethane.

6. The method according to claim 5, wherein the catheter (12) is reinforced with a structural member that is stiffer than the polymeric material of the catheter.

7. The method according to claim 1 or claim 2, wherein the removing step occurs while maintaining the catheter (12) intact.

8. The method according to claim 2, wherein the catheter inner surface is coated with a hydrophilic material that is the same as the hydrophilic material coated on the stylette (10).

9. The method according to claim 2, wherein the catheter inner surface is coated with a hydrophilic material that is different than the hydrophilic material coated on the stylette (10).

10. A combination of:
a catheter (12) having proximal and distal ends and an inner surface that is coated with polytetrafluoroethylene;
a hydrophilic coated stylette (10) within the catheter (12); and
a syringe (18) connected to the proximal end of the catheter (12) for wetting the hydrophilic coated stylette (10).

11. A combination of:
a catheter (12) having proximal and distal ends and an inner surface that is coated with a hydrophilic material;
a hydrophilic coated stylette (10) within the catheter (12); and
a syringe (18) connected to the proximal end of the catheter (12) for wetting the hydrophilic coated stylette (10).

12. The combination of claim 10 or claim 11, wherein the syringe (18) is connected to a syringe connector (14) at the proximal end of the catheter (12).

13. The combination of claim 12, wherein the syringe (18) contains pure saline.

## Patentansprüche

1. Verfahren zur Entfernung eines hydrophilen beschichteten Stylets (10) aus einem implantierbaren Katheter (12), wobei das Verfahren folgende Schritte umfasst:
Bereitstellung eines implantierbaren Katheters (12) mit einer Innenfläche, die mit Polytetrafluorethylen beschichtet ist;
Platzierung des hydrophilen beschichteten Stylets (10) im Katheter (12);
Benetzung des hydrophilen beschichteten Stylets (10); und
Entfernung des hydrophilen beschichteten Stylets (10) aus dem Katheter (12).

2. Verfahren zur Entfernung eines hydrophilen beschichteten Stylets (10) aus einem implantierbaren Katheter (12), wobei das Verfahren folgende Schritte umfasst:
Bereitstellung eines implantierbaren Katheters (12) mit einer Innenfläche, die mit einem hydrophilen Material beschichtet ist;
Platzierung des hydrophilen beschichteten Stylets (10) im Katheter (12) ;
Benetzung des hydrophilen beschichteten Stylets (10); und
Entfernung des hydrophilen beschichteten Stylets (10) aus dem Katheter (12).

3. Verfahren nach Anspruch 1 oder Anspruch 2, worin der Benetzungsschritt nach dem Schritt der Platzierung des hydrophilen beschichteten Stylets (10) im Katheter (12) erfolgt.

4. Verfahren nach Anspruch 1 oder Anspruch 2, worin der Entfernungsschritt nach dem Benetzungsschritt erfolgt.

5. Verfahren nach Anspruch 1 oder Anspruch 2, worin der Katheter (12) aus einem Polymermaterial wie Silikon oder Polyurethan besteht.

6. Verfahren nach Anspruch 5, worin der Katheter (12) mit einem Strukturelement verstärkt ist, das steifer ist als das Polymermaterial des Katheters.

7. Verfahren nach Anspruch 1 oder Anspruch 2, worin der Entfernungsschritt erfolgt, während der Katheter (12) intakt gehalten wird.

8. Verfahren nach Anspruch 2, worin die Innenfläche des Katheters mit einem hydrophilen Material beschichtet ist, das dasselbe hydrophile Material ist, mit dem das Stylet (10) beschichtet ist.

9. Verfahren nach Anspruch 2, worin die Innenfläche des Katheters mit einem hydrophilen Material beschichtet ist, das sich von dem hydrophilen Material, mit dem das Stylet (10) beschichtet ist, unterscheidet.

10. Kombination aus:
einem Katheter (12) mit proximalen und distalen Enden und einer Innenfläche, die mit Polytetrafluorethylen beschichtet ist;
einem hydrophilen beschichteten Stylet (10) im Katheter (12); und
einer mit dem proximalen Ende des Katheters (12) verbundenen Spritze (18) zur Benetzung des hydrophilen beschichteten Stylets (10).

11. Kombination aus:
einem Katheter (12) mit proximalen und distalen Enden und einer Innenfläche, die mit einem hydrophilen Material beschichtet ist;
einem hydrophilen beschichteten Stylet (10) im Katheter (12); und
einer mit dem proximalen Ende des Katheters (12) verbundenen Spritze (18) zur Benetzung des hydrophilen beschichteten Stylets (10).

12. Kombination nach Anspruch 10 oder Anspruch 11, worin die Spritze (18) mit einem Spritzenkonnektor (14) am proximalen Ende des Katheters (12) verbunden ist.

13. Kombination nach Anspruch 12, worin die Spritze (18) reine Kochsalzlösung enthält.

## Revendications

1. Procédé pour retirer un stylet revêtu d'une substance hydrophile (10) hors d'un cathéter implantable (12), ledit procédé comprenant les étapes suivantes:
prévoir un cathéter implantable (12) présentant une surface intérieure qui est revêtue de polytétrafluoroéthylène;
placer le stylet revêtu d'une substance hydrophile (10) à l'intérieur du cathéter (12);
humidifier le stylet revêtu d'une substance hydrophile (10); et
retirer le stylet revêtu d'une substance hydrophile (10) hors du cathéter (12).

2. Procédé pour retirer un stylet revêtu d'une substance hydrophile (10) hors d'un cathéter implantable (12), ledit procédé comprenant les étapes suivantes :
prévoir un cathéter implantable (12) présentant une surface intérieure qui est revêtue d'une substance hydrophile;
placer le stylet revêtu d'une substance hydrophile (10) à l'intérieur du cathéter (12);
humidifier le stylet revêtu d'une substance hydrophile (10); et
retirer le stylet revêtu d'une substance hydrophile (10) hors du cathéter (12).

3. Procédé selon la revendication 1 ou la revendication 2, dans lequel l'étape d'humidification est exécutée après l'étape de mise en place du stylet revêtu d'une substance hydrophile (10) à l'intérieur du cathéter (12).

4. Procédé selon la revendication 1 ou la revendication 2, dans lequel l'étape de retrait est exécutée après l'étape d'humidification.

5. Procédé selon la revendication 1 ou la revendication 2, dans lequel le cathéter (12) est constitué d'un matériau polymère, tel que le silicone ou le polyuréthane.

6. Procédé selon la revendication 5, dans lequel le cathéter (12) est renforcé avec un élément structurel qui est plus rigide que le matériau polymère du cathéter.

7. Procédé selon la revendication 1 ou la revendication 2, dans lequel l'étape de retrait est exécutée tout en maintenant le cathéter (12) intact.

8. Procédé selon la revendication 2, dans lequel la surface intérieure du cathéter est revêtue d'une substance hydrophile qui est la même que la substance hydrophile dont le stylet (10) est revêtu.

9. Procédé selon la revendication 2, dans lequel la surface intérieure du cathéter est revêtue d'une substance hydrophile qui est différente de la substance hydrophile dont le stylet (10) est revêtu.

10. Combinaison des éléments suivants:
un cathéter (12) qui présente des extrémités proximale et distale ainsi qu'une surface intérieure qui est revêtue de polytétrafluoroéthylène;
un stylet revêtu d'une substance hydrophile (10) à l'intérieur du cathéter (12); et
une seringue (18) qui est connectée à l'extrémité proximale du cathéter (12) pour humidifier le stylet revêtu d'une substance hydrophile (10).

11. Combinaison des éléments suivants:
un cathéter (12) qui présente des extrémités proximale et distale ainsi qu'une surface intérieure qui est revêtue d'une substance hydrophile;
un stylet revêtu d'une substance hydrophile (10) à l'intérieur du cathéter (12); et
une seringue (18) qui est connectée à l'extrémité proximale du cathéter (12) pour humidifier le stylet revêtu d'une substance hydrophile (10).

12. Combinaison selon la revendication 10 ou la revendication 11, dans laquelle la seringue (18) est connectée à un connecteur de seringue (14) à l'extrémité proximale du cathéter (12).

13. Combinaison selon la revendication 12, dans laquelle la seringue (18) contient une solution saline pure.
